Europäisches Patentamt

European Patent Office

Office européen des brevets

(11) Veröffentlichungsnummer: **0 486 582 B1**

(12) **EUROPÄISCHE PATENTSCHRIFT**

(45) Veröffentlichungstag der Patentschrift: **18.10.95**

(21) Anmeldenummer: **90912433.1**

(22) Anmeldetag: **31.07.90**

(86) Internationale Anmeldenummer:
**PCT/EP90/01248**

(87) Internationale Veröffentlichungsnummer:
**WO 91/01972 (21.02.91 91/05)**

(51) Int. Cl.⁶: $C07C$ **303/32**

(54) **VERFAHREN ZUR HERSTELLUNG VON HELLFARBIGEN ÖLSÄURESULFONATEN.**

(30) Priorität: **09.08.89 DE 3926344**

(43) Veröffentlichungstag der Anmeldung:
**27.05.92 Patentblatt 92/22**

(45) Bekanntmachung des Hinweises auf die
Patenterteilung:
**18.10.95 Patentblatt 95/42**

(84) Benannte Vertragsstaaten:
**AT BE CH DE DK ES FR GB IT LI NL SE**

(56) Entgegenhaltungen:
**GB-A- 1 278 421**

(73) Patentinhaber: **Henkel Kommanditgesellschaft auf Aktien**

**D-40191 Düsseldorf (DE)**

(72) Erfinder: **BEHLER, Ansgar**
**Siegfriedstrasse 80**
**D-4250 Bottrop (DE)**
Erfinder: **ANZINGER, Hermann**
**Emil-Barth-Strasse 1**
**D-4000 Düsseldorf 13 (DE)**
Erfinder: **VOGT, Michael**
**Ickeswaderstrasse 114**
**D-4000 Düsseldorf 13 (DE)**

**Beschreibung**

Die Erfindung betrifft ein verfahren zur Herstellung von hellfarbigen Ölsäuresulfonaten.

Die Herstellung von Ölsäuresulfonaten durch Umsetzung von technischen Ölsäureschnitten mit gasförmigem Schwefeltrioxid ist aus der GB-C 1 278 421 bekannt. Diese Veröffentlichung macht folgende Angaben über die Farbe der dabei erhaltenen Ölsäuresulfonate:

1. Die Farbe der Sulfonierungsprodukte ist abhängig von den Reaktionsbedingungen, insbesondere der Kontaktzeit im Reaktor und der Temperatur der dem Reaktor zugeführten Ausgangsprodukte.

2. Die Farbe der Sulfonierungsprodukte ist abhängig von der Menge an eingesetztem Schwefeltrioxid, wobei ein Überschuß von mehr als 15 Mol-%, bezogen auf Ölsäure, schlechtere Farben bei nur geringer Steigerung des Umsetzungsgrades ergeben.

3. Die Farbe der Sulfonierungsprodukte ist abhängig von dem Gehalt an polyungesättigten Fettsäuren in der eingesetzten technischen Ölsäure, wobei ein Gehalt von mehr als 3 % an diesen polyungesättigten Fettsäuren zu schlechten Farben der Sulfonierungsprodukte führt.

Es gibt auf dem Markt zahlreiche technische Ölsäurequalitäten, insbesondere solche aus nachwachsenden natürlichen Rohstoffen wie Rindertalg, Schweineschmalz, Olivenöl, Sonnenblumenöl und Palmkernöl, die Gehalte an polyungesättigten Fettsäuren von mehr als 3 % aufweisen und die bisher nicht nach dem aus der GB-PC 1 278 421 bekannten Verfahren unter Bildung hellfarbiger Ölsäuresulfonate sulfoniert werden konnten. Somit ist das Verfahren der Erfindung auf die Herstellung hellfarbiger Ölsäuresulfonate gerichtet, die sich aus technischen Ölsäurequalitäten mit höherem Gehalt an polyungesättigten Fettsäuren unter Verwendung von gasförmigem Schwefeltrioxid als Sulfonierungsmittel erhalten lassen.

Das Verfahren der Erfindung umfaßt die folgenden Stufen:

a) kontinuierliche Sulfonierung einer technischen Ölsäure mit einem Ölsäuregehalt von 65 bis 85 Gew.-% und einem Linolsäuregehalt bis zu 13 Gew.-%, Rest (auf 100 Gew.-%), andere gesättigte oder ungesättigte Fettsäuren mit 10 bis 22 Kohlenstoffatomen, mit gasförmigem Schwefeltrioxid in einem Molverhältnis von in der technischen Ölsäure vorhandenen olefinischen Doppelbindungen zu Schwefeltrioxid von 1 : 0,8 bis 1,0;

b) Neutralisation und Hydrolyse des in Stufe a) erhaltenen sauren Sulfonierungsproduktes mit wäßrigen Basen und

c) Bleichung des in Stufe b) als wäßrige Lösung erhaltenen Ölsäuresulfonates sowie gegebenenfalls

d) Konzentrierung der in Stufe c) erhaltenen, wäßrigen Lösung des gebleichten Ölsäuresulfonates.

Die in dem Verfahren der Erfindung einzusetzende technische Ölsäure enthält andere gesättigte oder ungesättigte Fettsäuren mit 10 bis 22 Kohlenstoffatomen, d.h. Fettsäuren, die nicht Ölsäure und Linolsäure sind und die üblicherweise in technischen Ölsäurequalitäten als Begleitstoffe enthalten sind, z.B. Caprin-, Laurin-, Myristin-, Myristolein-, Palmitin-, Palmitolein-, Stearin-, Linolen-, Arachin-, Gadolein-, Behen- und Erucasäure. Zweifach und mehr als zweifach ungesättigte Fettsäuren, die nicht Linolsäure sind, können in einer Menge enthalten sein, die bevorzugt nicht mehr als 2 Gew.-% beträgt.

Gemäß dem Verfahren der Erfindung wird das in der o.g. Stufe a) erhaltene saure Sulfonierungsprodukt mit wäßrigen Basen neutralisiert und hydrolysiert. Dabei reagieren die bei der Sulfonierungsreaktion gebildeten Sulfonsäuregruppen unter Bildung der entsprechenden Salze; weiterhin werden im Verlauf der Sulfonierung möglicherweise gebildete Sultone hydrolysiert. Typische Beispiele für die in der Neutralisation und Sulfonierung einsetzbaren wäßrigen Basen sind wäßrige Lösungen Hydroxiden von Alkalimetallen wie Natrium, Kalium oder Lithium von Oxiden oder Hydroxiden oder von Erdalkalimetallen wie Magnesium oder Calcium, Ammoniak oder organischer Basen wie Ethanolamin, Diethanolamin oder Triethanolamin. Im allgemeinen ist die Verwendung von wäßriger Natronlauge bevorzugt.

Gemäß dem Verfahren der Erfindung wird das in Stufe b) erhaltene Ölsäuresulfonat gebleicht; hierbei können die bei der Bleichung oberflächenaktiver Sulfonate und Sulfate üblichen Methoden verwendet werden. Es ist möglich und für Verfahren im technischen Maßstab auch vorteilhaft, die Stufen der Neutralisation und Hydrolyse sowie des Bleichens gleichzeitig durchzuführen. Das gebleichte Reaktionsprodukt kann anschließend, falls erwünscht, durch Entfernung von Wasser, z.B. durch Destillation, konzentriert werden.

Es war aufgrund des eingangs diskutierten Standes der Technik nicht zu erwarten gewesen, daß die Sulfonierung von technischen Ölsäurequalitäten mit einem Linolsäuregehalt bis zu 13 Gew.-%, insbesondere im Bereich von 4 bis 12 Gew.-%, bezogen auf das Gesamtgewicht der technischen Ölsäure, die Herstellung von bleichbaren Ölsäuresulfonaten ermöglicht.

Gemäß einer bevorzugten Ausführungsform des Verfahrens der Erfindung sulfoniert man eine technische Ölsäure, die einen Linolsäuregehalt im Bereich von 4 bis 13 Gew.-%, bezogen auf das Gesamtgewicht der technischen Ölsäure, aufweist.

Gemäß einer weiteren vorteilhaften Ausführungsform der Erfindung sulfoniert man die technische Ölsäure mit dem gasförmigen Schwefeltrioxid in einem Molverhältnis von in der technischen Ölsäure vorhandenen olefinischen Doppelbindungen zu Schwefeltrioxid im Bereich von 1 : 0,90 bis 0,98. Innerhalb dieses Bereiches lassen sich nach dem Bleichen besonders hell gefärbte Ölsäuresulfonate erhalten.

Die Sulfonierungsstufe des Verfahrens der Erfindung kann in den üblichen Reaktoren für die Umsetzung geeigneter Reaktanden mit gasförmigem Schwefeltrioxid durchgeführt werden; derartige Reaktoren sind z.B. in G. Dieckelmann, H.J. Heinz, The Basics of Industrial Oleochemistry, Peter Pomp GmbH, S. 111 bis 128 (1988) beschrieben. Die Sulfonierung in Reaktoren vom Typ der Fallfilmreaktoren ist bevorzugt, wobei es vorteilhaft ist, für die Sulfonierung ein 1 bis 10 Vol.-% Schwefeltrioxid enthaltendes inertes Gas zu verwenden. Bevorzugte Bedingungen für die Sulfonierung sind Reaktionstemperaturen im Bereich von 40 bis 80, insbesondere von 40 bis 60 °C.

Gemäß einer weiteren vorteilhaften Ausführungsform der Erfindung neutralisiert und hydrolysiert man die sauren Sulfonierungsprodukte bei Temperaturen im Bereich von 60 bis 95 °C, insbesondere 80 bis 90 °C, vorzugsweise innerhalb von 1 bis 8 Stunden und bei pH-Werten im Bereich von 5 bis 9.

Gemäß einer weiteren vorteilhaften Ausführungsform des Verfahrens der Erfindung bleicht man das neutralisierte und hydrolysierte Sulfonierungsprodukt mit wäßriger Wasserstoffperoxidlösung. Dabei können Lösungen mit einem Gehalt von 20 bis 90 Gew.-% Wasserstoffperoxid verwendet werden. Im allgemeinen setzt man die Wasserstoffperoxidlösung in einer Menge von 1 bis 4 Gew.-% Wasserstoffperoxid, bezogen auf Aktivsubstanzgehalt in dem neutralisierten und hydrolysierten Sulfonierungsprodukt, ein. Die Bleichung wird bevorzugt im pH-Bereich von 5 bis 9 durchgeführt.

Weiterhin hat es sich zum Erhalt hellfarbiger Ölsäuresulfonate als besonders vorteilhaft erwiesen, wenn man als Ausgangsprodukt eine destillierte technische Ölsäure einsetzt, die z.B. aus natürlichen Fetten und Ölen durch Umnetztrennung in Kombination mit einer vorhergehenden oder nachfolgenden Fettspaltung erhalten worden ist, und die nach der Destillation höchstens 1 Monat unter Luftzutritt gelagert worden ist.

Mittels des Verfahrens der Erfindung lassen sich technische Ölsäuresulfonate herstellen, die nach dem Bleichen Klettfarbzahlen von höchstens 100, insbesondere von höchstens 50, aufweisen.

Die Erfindung beruht somit auf der Erkenntnis, daß die folgenden Maßnahmen - einzeln oder in Kombination miteinander - zum Erhalt von hellfarbigen Ölsäuresulfonaten durch Sulfonierung einer technischen Ölsäure mit gasförmigem Schwefeltrioxid, Neutralisation und Hydrolyse sowie Bleichung von Bedeutung sind:

- ein Molverhältnis von technischer Ölsäure zu Schwefeltrioxid von 1 : 0,8 bis 1,0, insbesondere von 1 : 0,9 bis 0,98,
- ein Linolsäuregehalt von maximal 13 Gew.-%, insbesondere von 4 bis 12 Gew.-%;
- eine möglichst frisch destillierte Ölsäurequalität, die nach der Destillation nicht länger als 1 Monat unter Luftzutritt gelagert sein soll;
- eine Bleichung mit wäßrigen Wasserstoffperoxidlösungen in einer Menge entsprechend 0,5 bis 4, insbesondere 1,0 bis 2 Gew.-% Wasserstoffperoxid, bezogen auf aktive Substanz in den erhaltenen wäßrigen Lösungen des Ölsäuresulfonates.

Die Erfindung wird im folgenden anhand von bevorzugten Ausführungsbeispielen näher erläutert, wobei auf die am Schluß der Beschreibung befindliche Tabelle Bezug genommen wird.

Die Tabelle 1 enthält für 7 verschiedene, erfindungsgemäß einzusetzende, handelsübliche technische Ölsäurequalitäten die gaschromatografisch ermittelten Zusammensetzungen (in Flächenprozent) und die Säurezahl (SZ) sowie die Jodzahl (JZ); weiterhin die Klett-Farbzahl nach dem Bleichen. Weiterhin enthält die Tabelle die entsprechenden Werte für eine zu Vergleichszwecken eingesetzte Ölsäure A mit einem außerhalb des beanspruchten Bereiches liegenden Linolsäuregehalt.

Die in den eingesetzten technischen Ölsäurequalitäten ermittelten Fettsäuren sind in einer verkürzten Schreibweise wiedergegeben. Die tiefgestellte Zahl nach dem Buchstaben C bedeutet die Zahl der Kohlenstoffatome in der Kette; die Anzahl der Striche die Zahl der in der Kette vorhandenen olefinischen Doppelbindungen. So bedeutet z.B. $C_{18}$ Stearinsäure, $C_{18}'$ Ölsäure, $C_{18}''$ Linolsäure und $C_{18}'''$ Linolensäure.

Die eingesetzten Ölsäurequalitäten Nr. 1 bis 7 sowie A waren folgender Herkunft:

Nr. 1:  aus Rindertalg
Nr. 2:  aus Olivenöl
Nr. 3:  aus Sonnenblumenöl
Nr. 4:  aus Palmkernöl
Nr. 5:  aus Olivenöl
Nr. 6:  aus Rindertalg
Nr. 7:  aus Rindertalg

EP 0 486 582 B1

A :     aus Rapsöl.

Die vorgenannten Ölsäurequalitäten sind im Handel erhältliche Produkte.

Allgemeine Arbeitsvorschrift für die Sulfonierung von technischer Ölsäure.

Die Sulfonierungen wurden in einem Fallfilmreaktor aus Glas vorgenommen, der im wesentlichen aus einem von einem Heiz- und Kühlmantel umgebenen Rohr mit einer Länge von 1100 mm und einem Innendurchmesser von 6 mm bestand. Der Reaktor war am Kopf mit einer Aufgabevorrichtung für die Ölsäure und mit einem Gaseinleitungsrohr versehen. Gasförmiges Schwefeltrioxid, erzeugt durch Erhitzen von Oleum, wurde mit Stickstoff auf eine Konzentration von 5 Vol.-% Schwefeltrioxid verdünnt und für die Sulfonierung eingesetzt.

Die Ölsäure wurde mit einer konstanten Geschwindigkeit von 550 g/h aufgegeben. Die Zufuhr des Schwefeltrioxid/Stickstoff-Gemisches wurde so eingestellt, daß das Molverhältnis von in der technischen Ölsäure vorhandenen olefinischen Doppelbindungen (errechnet aus der Jodzahl) zu Schwefeltrioxid 1 : 0,90 betrug. Mit Hilfe eines Wasserkreislaufs durch den Reaktormantel wurde die Reaktionstemperatur der Sulfonierung bei 50 ° C gehalten.

Nach dem Verlassen des Reaktors wurde das Reaktionsgemisch in einem Becherglas, das 25 gew.-%-ige Natronlauge enthielt, aufgefangen und anschließend bei einem pH-Wert von 8 bis 9 zwei Stunden lang auf 90 ° C erhitzt.

Das hydrolysierte Produkt wurde bei Umgebungstemperatur (ca. 20 ° C) portionsweise mit zwei Gew.-% Wasserstoffperoxid, bezogen auf aktive Substanz, zugesetzt in Form einer 35 Gew.-%-igen wäßrigen Wasserstoffperoxidlösung, versetzt.

Es wurden gebleichte, hellgelbe, in Wasser klargelöste Produkte erhalten. Das mit der Ölsäure Nr. 4 gemäß Tabelle 1 erhaltenen Produkt wies die folgenden Kennzahlen auf:

| | |
|---|---|
| waschaktive Substanz (nach Epton*; MG = 405): | 37,0 % |
| unsulfonierbare Substanz (Petrolether-Extrakt): | 4,5 % |
| Natriumsulfat: | 1,1 % |
| Trockenrückstand: | 46,1 % |
| Klett-Farbzahl (1 cm-Rundküvette): | 12 |

*) siehe Nature 160 (1947), 759 und Tenside 4 (1967), 292.

Die Bestimmung der Klett-Farbzahl erfolgte in einem Klett-Summerson-Photometer mit Blaufilter (400 bis 450 nm) unter Verwendung von 1 cm-Rundküvetten an einer wäßrigen Lösung, die 5 Gew.-% waschaktive Substanz enthält.

Die mit den oben genannten Ölsäurequalitäten bei unterschiedlichen Lagerzeiten und verschiedenen Molverhältnissen von in dem eingesetzten Ölsäuregemisch vorhandenen Doppelbindungen zu Schwefeltrioxid erhaltenen Klett-Farbzahlen sind in Tabelle 2 am Schluß der Beschreibung zusammengefaßt. Es zeigt sich aus den Versuchen mit der Ölsäure Nr. 6, daß eine Lagerzeit von sechs Monaten im Vergleich zu einer frisch destillierten Ölsäure (Lagerzeit = 0 Monate) zu erheblich schlechteren Klett-Farbzahlen führt. Bereits eine Lagerzeit von mehr als einem Monat (unter Luftzutritt) führt zu einer merklichen Verschlechterung der Klettfarbzahl. Wie sich aus dem Versuch mit der technischen Ölsäure A ergibt, liefern Ölsäurequalitäten mit einem Linolsäuregehalt von über 13 % Ölsäuresulfonate, die nicht mehr bleichbar sind.

4

## Tabelle 1

### Eingesetzte technische Ölsäuren

| Ölsäure Nr. Zusammensetzung | 1 | 2 | 3 | 4 | 5 | 6 | 7 | A |
|---|---|---|---|---|---|---|---|---|
| $C_{12}$ | 0,1 | | | 0,5 | | 0,4 | 0,2 | - |
| $C_{14}$ | 2,7 | 0,1 | <0,5 | 1,2 | | 2,1 | 2,6 | |
| $C_{14}'$ | 0,8 | | | | | 0,3 | 2,4 | 0,5 |
| $C_{15}$ | 0,5 | | | | | 0,4 | 0,4 | - |
| $C_{16}$ | 4,1 | 16,2 | 3,5 | 4,3 | 4,6 | 4,6 | 3,7 | 5,5 |
| $C_{16}'$ | 5,4 | 1,1 | <1 | 0,3 | 1,0 | 5,0 | 10,3 | |
| $C_{17}$ | 1,3 | 0,2 | <1 | 0,4 | 0,2 | 1,2 | 2,5 | |
| $C_{18}$ | 1,0 | 2,3 | 3,6 | 1,0 | 1,6 | 0,9 | 0,9 | 1,5 |
| $C_{18}'$ | 73,3 | 68,7 | 82,8 | 80,0 | 82,7 | 70,7 | 72,6 | 56 |
| $C_{18}''$ | 8,7 | 10,1 | 8,4 | 11,0 | 8,1 | 11,8 | 2,7 | 21,5 |
| $C_{18}'''$ | 1,0 | 0,5 | <1 | 0,2 | 0,7 | 0,6 | 0,5 | 10,5 |
| $C_{20}$ | 0,1 | 0,3 | 0,1 | 0,2 | 0,3 | 0,2 | 0,2 | 0,5 |
| $C_{20}'$ | 1,1 | 0,4 | <1 | 0,2 | 0,3 | 1,8 | 1,0 | 1,5 |
| SZ | 201,7 | 200,0 | 196,3 | 199,8 | 194,7 | 200,4 | 202,7 | 199 |
| JZ | 93,5 | 85,1 | 86,5 | 92,5 | 92,3 | 93,8 | 85,2 | 122 |

EP 0 486 582 B1

EP 0 486 582 B1

Tabelle 2

| Einfluß von Lagerzeiten und Molverhältnissen auf die Klett-Farbzahl | | | | |
|---|---|---|---|---|
| aus Ölsäure Nr. | Lagerzeit (Monate) | Molverhältnis C = C : SO$_3$ | Sulfiergrad (%) | Klett-Farbzahl |
| 1 | 0 | 0,98 | 86,4 | 36 |
| 1 | 1 | 1,0 | 90,2 | 38 |
| 1 | 2 | 0,90 | 87,1 | 46 |
| 2 | 0 | 0,94 | 81,5 | 21 |
| 3 | 0 | 0,94 | 85,7 | 19 |
| 4 | 0 | 0,97 | 90,1 | 39 |
| 4 | 0 | 0,94 | 86,2 | 12 |
| 5 | 0 | 0,91 | 85,0 | 23 |
| 6 | 0 | 0,92 | 87,0 | 45 |
| 6 | 6 | 0,90 | 84,5 | 98 |
| 6 | 6 | 0,97 | 88,6 | 165 |
| 7 | 0 | 0,94 | 83,5 | 10 |
| A | 0 | 0,94 | 90,5 | >600 |

## Patentansprüche

1. Verfahren zur Herstellung von hellfarbigen Ölsäuresulfonaten mit den Stufen
   a) kontinuierliche Sulfonierung einer technischen Ölsäure mit einem Ölsäuregehalt von 65 bis 85 Gew.-% und einem Linolsäuregehalt bis zu 13 Gew.-%, Rest (auf 100 Gew.-%), andere gesättigte oder ungesättigte Fettsäuren mit 10 bis 22 Kohlenstoffatomen, mit gasförmigem Schwefeltrioxid in einem Molverhältnis von in der technischen Ölsäure vorhandenen olefinischen Doppelbindungen zu Schwefeltrioxid von 1 : 0,8 bis 1,0,
   b) Neutralisation und Hydrolyse des in Stufe a) erhaltenen sauren Sulfonierungsproduktes mit wäßrigen Basen und
   c) Bleichung des in Stufe b) als wäßrige Lösung erhaltenen Ölsäuresulfonates
   sowie gegebenenfalls
   d) Konzentrierung der in Stufe c) erhaltenen wäßrigen Lösung des Ölsäuresulfonates.

2. Verfahren nach Anspruch 1, dadurch gekennzeichnet, daß man eine technische Ölsäure sulfoniert, die einen Linolsäuregehalt im Bereich von 4 bis 13 Gew.-%, bezogen auf das Gesamtgewicht der technischen Ölsäure, aufweist.

3. Verfahren nach Anspruch 1 oder 2, dadurch gekennzeichnet, daß man die technische Ölsäure mit dem gasförmigen Schwefeltrioxid in einem Molverhältnis von in der technischen Ölsäure vorhandenen olefinischen Doppelbindungen zu Schwefeltrioxid im Bereich von 1 : 0,90 bis 1 : 0,98 sulfoniert.

4. Verfahren nach mindestens einem der Ansprüche 1 bis 3, dadurch gekennzeichnet, daß man die Sulfonierung in einem Fallfilmreaktor durchführt.

5. Verfahren nach mindestens einem der Ansprüche 1 bis 4, dadurch gekennzeichnet, daß man für die Sulfonierung ein 1 bis 10 Vol-% Schwefeltrioxid enthaltendes, inertes Gas verwendet.

6. Verfahren nach mindestens einem der Ansprüche 1 bis 5, dadurch gekennzeichnet, daß man die Sulfonierung bei Temperaturen im Bereich von 40 bis 80, insbesondere 40 bis 60 °C, durchführt.

7. Verfahren nach mindestens einem der Ansprüche 1 bis 6, dadurch gekennzeichnet, daß man die sauren Sulfonierungsprodukte bei Temperaturen im Bereich von 60 bis 95 °C, insbesondere von 80 bis 90 °C, neutralisiert und hydrolysiert.

8. Verfahren nach mindestens einem der Ansprüche 1 bis 7, dadurch gekennzeichnet, daß man die sauren Sulfonierungsprodukte innerhalb von 1 bis 8 Stunden neutralisiert und hydrolysiert.

6

9. Verfahren nach mindestens einem der Ansprüche 1 bis 8, dadurch gekennzeichnet, daß man die sauren Sulfonierungsprodukte bei pH-Werten im Bereich von 5 bis 9 neutralisiert und hydrolysiert.

10. Verfahren nach mindestens einem der Ansprüche 1 bis 9, dadurch gekennzeichnet, daß man die sauren Sulfonierungsprodukte mit wäßriger Alkalilauge neutralisiert und hydrolysiert.

11. Verfahren nach mindestens einem der Ansprüche 1 bis 10, dadurch gekennzeichnet, daß man das als wäßrige Lösung erhaltene Ölsäuresulfonat mit wäßriger Wasserstoffperoxidlösung bleicht.

12. Verfahren nach mindestens einem der Ansprüche 1 bis 11, dadurch gekennzeichnet, daß man die Bleichung des Ölsäuresulfonates bei pH-Werten im Bereich von 5 bis 9 durchführt.

13. Verfahren nach mindestens einem der Ansprüche 1 bis 12, dadurch gekennzeichnet, daß man eine destillierte technische Ölsäure verwendet, die nach der Destillation höchstens 1 Monat unter Luftzutritt gelagert worden ist.

14. Verfahren nach mindestens einem der Ansprüche 1 bis 13, dadurch gekennzeichnet, daß man technische Ölsäuresulfonate herstellt, die nach dem Bleichen Klettfarbzahlen von höchstens 100, insbesondere von höchstens 50, aufweisen.

**Claims**

1. A process for the preparation of a light-colored oleic acid sulfonate with the stages
   a) continuous sulfonation of an industrial oleic acid having an oleic acid content of 65 to 85% by weight and a linoleic acid content of up to 13% by weight, the remainder (to 100% by weight) being other saturated or unsaturated fatty acids having 10 to 22 carbon atoms, using gaseous sulfur trioxide in a molar ratio of olefinic double bonds present in the industrial oleic acid to sulfur trioxide of 1 : 0.8 to 1.0,
   b) neutralization and hydrolysis of the acid sulfonation product obtained in stage a) with aqueous bases and
   c) bleaching of the oleic acid sulfonate obtained in stage b) as an aqueous solution, and if appropriate
   d) concentration of the aqueous solution, obtained in stage c), of the oleic acid sulfonate.

2. The process as claimed in claim 1, wherein an industrial oleic acid which has a linoleic acid content in the range from 4 to 13% by weight, based on the total weight of the industrial oleic acid, is sulfonated.

3. The process as claimed in claim 1 or 2, wherein the industrial oleic acid is sulfonated with the gaseous sulfur trioxide in a molar ratio of olefinic double bonds present in the industrial oleic acid to sulfur trioxide in the range from 1 : 0.90 to 1 : 0.98.

4. The process as claimed in at least one of claims 1 to 3, wherein the sulfonation is carried out in a falling film reactor.

5. The process as claimed in at least one of claims 1 to 4, wherein an inert gas containing 1 to 10% by volume of sulfur trioxide is used for the sulfonation.

6. The process as claimed in at least one of claims 1 to 5, wherein the sulfonation is carried out at a temperature in the range from 40 to 80, in particular 40 to 60 °C.

7. The process as claimed in at least one of claims 1 to 6, wherein the acid sulfonation product is neutralized and hydrolyzed at a temperature in the range from 60 to 95 °C, in particular from 80 to 90 °C.

8. The process as claimed in any one of claims 1 to 7, wherein the acid sulfonation product is neutralized and hydrolyzed in the course of 1 to 8 hours.

9. The process as claimed in any one of claims 1 to 8, wherein the acid sulfonation product is neutralized and hydrolyzed at a pH in the range from 5 to 9.

10. The process as claimed in any one of claims 1 to 9, wherein the acid sulfonation product is neutralized and hydrolyzed with an aqueous alkali metal hydroxide solution.

11. The process as claimed in any one of claims 1 to 10, wherein the oleic acid sulfonate obtained as an aqueous solution is bleached with aqueous hydrogen peroxide solution.

12. The process as claimed in any one of claims 1 to 11, wherein the bleaching of the oleic acid sulfonate is carried out at a pH in the range from 5 to 9.

13. The process as claimed in any one of claims 1 to 12, wherein a distilled industrial oleic acid which has been stored for not more than 1 month in contact with air after the distillation is used.

14. The process as claimed in any one of claims 1 to 13, wherein an industrial oleic acid sulfonate which has a Klett color number of not more than 100, in particular of not more than 50, after bleaching is prepared.

**Revendications**

1. Procédé pour la préparation de sulfonates d'acide oléique de couleur claire, comprenant les étapes de :
   a) sulfonation en continu d'un acide oléique technique possédant une teneur en acide oléique de 65 à 85% en poids et une teneur en acide linoléique jusqu'à 13% en poids, le reste (sur 100% en poids) étant constitué par d'autres acides gras saturés ou insaturés contenant de 10 à 22 atomes de carbone, avec du trioxyde de soufre gazeux dans un rapport molaire des doubles liaisons oléfiniques présentes dans l'acide oléique technique au trioxyde de soufre de 1:0,8 à 1,0;
   b) neutralisation et hydrolyse avec des bases aqueuses du produit de sulfonation acide obtenu à l'étape (a); et
   c) blanchiment du sulfonate d'acide oléique obtenu à l'étape b) sous forme d'une solution aqueuse, de même que, le cas échéant,
   d) concentration de la solution aqueuse du sulfonate d'acide oléique obtenue à l'étape c).

2. Procédé selon la revendication 1, caractérisé en ce qu'on soumet à une sulfonation, un acide oléique technique qui présente une teneur en acide linoléique dans le domaine de 4 à 13% en poids, rapportée au poids total de l'acide oléique technique.

3. Procédé selon la revendication 1 ou 2, caractérisé en ce qu'on soumet à une sulfonation l'acide oléique technique avec le trioxyde de soufre gazeux dans un rapport molaire des doubles liaisons oléfiniques présentes dans l'acide oléique technique au trioxyde de soufre dans le domaine de 1:0,90 à 1:0,98.

4. Procédé selon au moins une des revendications 1 à 3, caractérisé en ce qu'on effectue la sulfonation dans un réacteur à film tombant.

5. Procédé selon au moins une des revendications 1 à 4, caractérisé en ce qu'on utilise, pour la sulfonation, un gaz inerte contenant de 1 à 10% en volume de trioxyde de soufre.

6. Procédé selon au moins une des revendications 1 à 5, caractérisé en ce qu'on effectue la sulfonation à des températures dans le domaine de 40 à 80, en particulier de 40 à 60°C.

7. Procédé selon au moins une des revendications 1 à 6, caractérisé en ce qu'on soumet à une neutralisation et à une hydrolyse les produits de sulfonation acides, à des températures dans le domaine de 60 à 95°C, en particulier de 80 à 90°C.

8. Procédé selon au moins une des revendications 1 à 7, caractérisé en ce qu'on soumet à une neutralisation et à une hydrolyse les produits de sulfonation acides dans un laps de temps de 1 à 8 heures.

9. Procédé selon au moins une des revendications 1 à 8, caractérisé en ce qu'on soumet à une neutralisation et à une hydrolyse les produits de sulfonation acides à des valeurs de pH dans le domaine de 5 à 9.

10. Procédé selon au moins une des revendications 1 à 9, caractérisé en ce qu'on soumet à une neutralisation et à une hydrolyse les produits de sulfonation acides avec de la lessive alcaline aqueuse.

11. Procédé selon au moins une des revendications 1 à 10, caractérisé en ce qu'on soumet à un blanchiment le sulfonate d'acide oléique obtenu sous forme d'une solution aqueuse, avec une solution aqueuse de peroxyde d'hydrogène.

12. Procédé selon au moins une des revendications 1 à 11, caractérisé en ce qu'on effectue le blanchiment du sulfonate d'acide oléique à des valeurs de pH dans le domaine de 5 à 9.

13. Procédé selon au moins une des revendications 1 à 12, caractérisé en ce qu'on utilise un acide oléique technique distillé, qui a été entreposé après la distillation au maximum pendant 1 mois sous apport d'air.

14. Procédé selon au moins une des revendications 1 à 13, caractérisé en ce qu'on prépare des sulfonates d'acide oléique technique qui présentent, après le blanchiment, des indices de couleurs de Klett d'au maximum 100, en particulier d'au maximum 50.